# EUROPEAN PATENT SPECIFICATION

(11) **EP 0 367 886 B1**
(45) Date of publication and mention of the grant of the patent: **19.04.1995**
(21) Application number: 88810717.4
(22) Date of filing: 19.10.1988
(51) Int. Cl.: C07C 233/09, C08F 20/58, C07K 1/26, C07K 1/16, C12P 19/34

(54) **Hydrophilic monomers, polymers thereof and their applications**
Hydrophile Monomere, Polymere davon und ihre Verwendungen
Monomères hydrophiles, ses polymères et leurs utilisations

(43) Date of publication of application: 16.05.1990
(73) Proprietor: Guest Elchrom Scientific AG, CH-6330 Cham (CH)
(72) Inventor: Kozulic, Branko, Dr., CH-8046 Zürich (CH)
(74) Representative: Frei, Alexandra Sarah

(56) References cited:
- EP-A- 0 227 054
- FR-A- 934 879
- FR-A- 2 162 724
- US-A- 3 898 279
- MAKROMOLECULAR CHEMIE, vol. 188, no. 6, June 1987, pages 1217-1232; J. KLEIN et al, "Synthesis of some poly(vinylsaccharide)s of the amide type and investigation of their solution properties"
- JOURNAL OF ORGANIC CHEMISTRY, vol. 26, May 1961, pages 1583-1588; R.L. WHISTLER et al, "1-Acrylamido-1-deoxy-D-glucitol, 1-deoxy-1-methacrylamido-D-glucitol and their polymerization"
- CHEMICAL ABSTRACTS, vol. 85, no. 2, 12th July 1976, page 1, abstract no. 6081d,Columbus, Ohio, US; J. PAPROTNY et al.: "Synthesis and polymerization of N-substituted acrylamides. Part V. Acrylamides, derivatives of 2,3-dihydroxypropylamine"

## Description

This invention concerns acrylic monomers, polymers made of these monomers and applications of the cross-linked polymers.

### BACKGROUND OF THE INVENTION

There are two most important techniques for separation of biomolecules. Chromatography is generally used for a preparative purification of biological molecules, whereas electrophoresis is the most powerful technique for analysis of the molecules in crude samples and at various stages of the purification procedure.

Each of these two techniques uses separation media of unique properties. Nevertheless, media for chromatography and electrophoresis can be prepared from the same starting material. Until now, two acrylic monomers have been successfully used to prepare gels for electrophoresis and chromatography. These two monomers are acrylamide and N-acryloyl-tris(hydroxymethyl)-aminomethane (NAT). The inventor of the present invention is one of the persons who has introduced the NAT-monomer to prepare gels for electrophoresis (References 1-3).

The poly-NAT gels possessed several advantages over the polyacrylamide gels (References 1-3). In addition to their pronounced hydrophilicity, the most important advantage appeared to be a higher porosity of these gels. Since a gel even more hydrophilic and porous than a poly-NAT gel would be beneficial in many applications, it was worth searching for a monomer which will produce such a gel.

A NAT solution has a molar concentration lower than the polyacrylamide solution of the same percentage, because the molecular weight of NAT is about 2.5 fold higher than the molecular weight of acrylamide. The poly-NAT gels were found to be approximately 3 times more porous than the corresponding polyacrylamide gels, which is in good agreement with the 2.5 fold lower molarity. Thus, one can assume that the lower molar concentration of NAT solutions results after polymerization in fewer polymer chains per unit volume, leading to gels of increased porosity. If this assumption is correct, then even more porous gels will be formed from monomers of higher molecular weight. However, the lack of mechanical strength may be a drawback of gels produced from monomers of very high molecular weight. The optimal properties are expected to be inherent to the monomers of medium size, due to a balanced ratio between the size of the polymer backbone and the size of the side chains present in every repeating unit.

In addition to the size of a monomer, other factors can influence the porosity of a gel. If interactions exist between monomer molecules, or between a monomer and a growing polymer, or between the two growing polymer chains, then as a result of these interactions the polymer chains will not be randomly distributed. They are likely to form some kind of bundles and thus create larger pores. It is generally accepted that the high porosity of agarose gels comes from the association of polymer chains during the gelation process. The resulting bundles of polymer chains are presumably held by hydrogen bonds. Other types of the stabilizing forces, such as hydrophobic interaction or ionic bonds, are not compatible with the media that are to be used for electrophoresis or chromatography.

From the above considerations, it seemed that monomers composed of sugar alcohols might form gels with desirable properties. They are hydrophilic, they are of medium size and they have 4-5 hydroxyl groups which could form hydrogen bonds. As shown in the present invention, many such compounds can be conveniently and controled synthesized. In addition, such synthesized compounds easily polymerize and form gels useful for electrophoresis and chromatography. Two special monomers of the type described in this invention, N-acryloyl-1-amino-1-deoxy-D-glucitol and N-methacryloyl-1-amino-1-deoxy-D-glucitol, have been already synthesized (Reference 4), but they were not used to prepare crosslinked gels for electrophoresis or chromatography.

### OBJECTIVES OF THE INVENTION

It is an object of the present invention to provide a process (method) for preparation of monomers such as N-acryloyl and N-methacryloylamino sugar alcohols.

It is another object of the present invention to provide a process (method) for preparation of polymers from these monomers, especially of such polymers that are suitable as media for electrophoresis and chromatography.

It is a further object of the invention to show the preparation of gel-systems for molecules separation techniques e.g. electrophoresis or chromatography using the inventive polymers.

### SUMMARY OF THE INVENTION

This invention concerns the monomers composed of amino sugar alcohols whose primary or secondary amino group has been derivatized (derived) by an acryloyl or an methacryloyl function. The amino group can be linked to any carbon of five or six-carbon sugar alcohols.

The general formula of the monomers is given below. 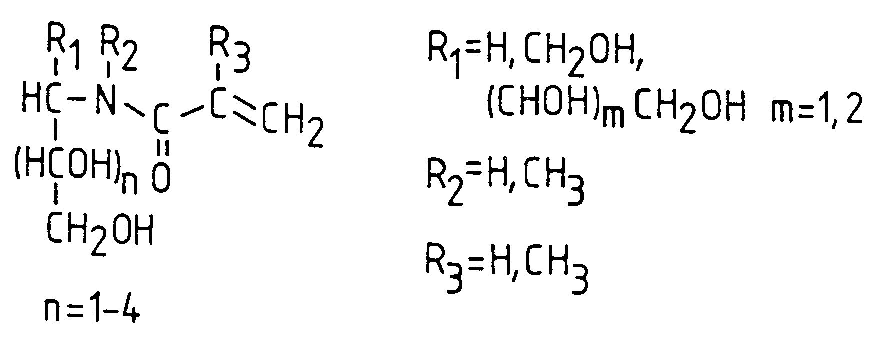
These monomers are synthesized by reacting the amino group of a sugar alcohol with an activated derivative of acrylic or methacrylic acid, such as acryloyl chloride or methacryloyl chloride, in a water-methylene chloride solvent two-phase mixture by alternate additions of the acid chloride and an alkali hydroxide at pH 7.5 - 9.5 and removing the charged by-products by a combination of an anionic and cationic ion exchanger.

The monomers represented by the above shown general formula have been polymerized by a free radical polymerization, either alone or with other compounds and materials having polymerizable double bonds.

The so formed polymers were linear or branched (cross-linked). The cross-linked polymers were formed when cross-linkers with two or more double bonds were present during polymerization, and in some cases when concentrated solutions of monomers were polymerized.

The cross-linked polymers prepared were usually in the form of gels. The gels obtained were opaque or transparent, depending on the type and amount of the cross-linker. The gels have been polymerized in different form and shapes, i.e., beads, thin sheets, rods, blocks, etc.

The most important application of these new types of gel was found in their use as separation media. The transparent gels were used as an anticonvective matrix in electrophoresis, whereas the opaque gels prepared in a bead form were used in chromatography.

### DETAILED DESCRIPTION OF THE INVENTION

Various aspects of the present invention are illustrated by 8 examples and 4 figures.

### Example 1.

### Synthesis of N-acryloyl-2-amino-2-deoxy-D-glucitol.

The starting material for preparation of this monomer is N-acetyl-2-amino-2-deoxy-D-glucose. The synthesis includes mainly three steps, which are described below.
**Step 1**. N-acetyl-2-amino-2-deoxy-D-glucose is first reduced to the corresponding glucitol. Thus 0.5 mol (110.6 g) of N-acetyl-2-amino-2-deoxy-D-glucose is dissolved in about 400 ml water and the solution is cooled in an ice bath. Then 0.2 mol (7.6 g) of sodium borohydride is dissolved in about 50 ml of 1 M sodium hydroxyde. This solution is added in small portions into the efficiently stirred sugar solution. The stirring process is continued for 2-4 h after all sodium borohydride had been added. At this time, there should be an excess of unreacted sodium boronhydride, which is checked by dropping one drop of the reaction solution into 50% acetic acid. If no hydrogen bubbles are generating (visible), some more NaBH₄ is added to the reaction mixture. (Note 1. This may be the case with too old NaBH₄ preparations). If hydrogen is liberated, the reaction mixture is treated with Amberlite IR-120, H⁺. The resin is added carefully (to prevent excessive foaming) in small portions, whilst stirring and cooling in the ice bath is continued. out 200-300 ml of the resin is added. After additional stirring, there is usually very little sodium left, as detected by burning a drop of solution on platinum wire. The remaining sodium is removed by passing the reaction mixture through a 100 ml column filled with Dowex 50, H⁺ (200-400 or 100-200 mesh). The reaction mixture, which contains now N-acetyl-2-amino-2-deoxy-D-glucitol and boric acid, is evaporated under reduced pressure at 30-40xC. The resulting syrup is mixed with about 200 ml of methanol-acetic acid solution (20:1, v/v) and evaporated. This procedure is repeated 4-5 times, and it serves to remove boric acid as trimethyl borate.
**Step 2**. The syrupy residue is dissolved in 500 ml of 2 M HCl and the solution is refluxed for 4-8 h. The solution is concentrated by evaporation and then dried in a desiccator over phosphorous pentoxide and potassium hydroxyde pellets. The product, 2-amino-2-deoxy-D-glucitol hydrochloride, can be obtained in the crystalline state but this is not necessary for the next step. The yield of the crude, yellow to brownish product ranged from 73 to 89% from four different preparations.
**Step 3**. The crude 2-amino-2-deoxy-D-glucitol hydrochloride is dissolved in about 300 ml of water and the pH of this solution is adjusted to 8.0-8.5 with 5 M KOH. Then 1-2 g of sodium nitrite and 100-150 ml of methylene chloride are added, and the solution is cooled in an ice bath. Acryloyl chloride (10% molar excess over 2-amino-2-deoxy-D-glucitol) is mixed with the same volume of methylene chloride. Potassium hydroxyde, equal to twice the molar amount of acryloyl chloride, is dissolved in water and cooled. To the well stirred two-phase reaction mixture, portions of acryloyl chloride and KOH solutions were added in such a way that the pH remained between 7.5-9.5 (as checked frequently by a narrow range pH paper). After the last additions of acryloyl chloride and KOH solutions, the reaction mixture was further stirred for about one hour (the pH was periodically checked and corrected, if necessary). The two phases were allowed to separate in a separating funnel and the lower organic phase was discarded. The aqueous phase was treated in one of the two ways. A fraction of salts was precipitated with addition of four volumes of absolute ethanol and then the remaining salts were bound to the mixed ion exchangers. Alternatively, the precipitation step was omitted and the salts were directly removed by mixed ion exchangers. To the stirred solution, portions of Amberlite IR-120, H⁺ and IRA-68 (free base) were added (pH being kept neutral or slightly acid) until the silver reaction for chloride was negative. The resin was then removed, the filtrate treated with activated charcoal and the solution poured into crystalization dishes. A small quantity (several hundred mg) of p-methoxy phenol (polymerization inhibitor) was added. The solvents evaporated within 1-2 weeks. The monomer appeared as a hard, slightly yellow but almost transparent residue. The yield ranged from 43-56% with regard to the starting material, N-acetyl-2-amino-2-deoxy-D-glucose. The product was pulverized and recrystallized from ethanol, m.p. 133-135 (polymerization).

**Note 2**. An attempt was done to prepare the same compound by reacting 2-amino-2-deoxy-D-glucose and acryloyl chloride, followed by reduction of the product with sodium borohydride. However, it was not possible to get pure N-acryloyl-2-amino-2-deoxy-D-glucitol in this way.

### Example 2.

### Synthesis of N-acryloyl-N-methyl-1-amino-1-deoxy-D-glucitol.

N-methyl-1-amino-1-deoxy-D-glucitol (0.5 mol, 97.6 g) was dissolved in about 300 ml of water. Then 1-2 g of sodium nitrite was added and the solution was cooled in an ice bath. Potassium hydroxyde (0.6 mole) was dissolved in water and cooled. Acryloyl chloride (0.55 mole) was mixed with an equal volume of methylene chloride. The reaction was done in the same way as described in step 3 for the synthesis of N-acryloyl-2-amino-2-deoxy-D-glucitol. The salts were also removed as described above. However, even after several week long evaporation from a crystallization dish, the product was not dry. After addition of more polymerization inhibitor, drying was continued in a desiccator. The semi-dry mass (yield 41-60%) was dissolved in warm dioxane which contained p-methoxy phenol. The warm solution was filtered through Celite and upon cooling the crystals were formed. They were collected and recrystallized from dioxane. The title compound is very hygroscopic.

### Example 3.

### Synthesis of N-methacryloyl-N-methyl-1-amino-1-deoxy-D-glucitol.

This compound was prepared in two slightly different ways. The first way is identical to the preparation of N-acryloyl-N-methyl-1-amino-1-deoxy-D-glucitol described above. In contrast to the previous one, this monomer readily crystallized upon drying in crystallization dishes (yield 62-80%). It was recrystallized from ethanol, m.p. 142-144.

A simpler way involves the use of lithium hydroxyde instead of potassium hydroxyde to neutralize hydrochloric acid that is formed during the reaction. After separation of the two phases, the water phase was without any treatment poured into crystalization dishes. After a few days in the fume cupboard, the title compound crystallized from the highly concentrated salt (mostly LiCl) solution. The crystals were collected and washed with ethanol. The yield was lower (about 50%).

### Example 4.

### Polymerization of N-acryloyl-2-amino-2-deoxy-D-glucitol.

The monomer (450 mg) was dissolved in water to give 4.5% (w/v) solution (l0 ml). To this solution were added 12 »l of a 50% (v/v) N,N,N′,N′-tetramethylethylendiamine (TEMED) solution, followed by 155 »l of ammonium persulfate solution (15 mg/ml). The monomer solution was ovelaid by diisopropyl ether. After 24 h at room temperature a highly viscous solution was produced. The polymerization of N-acryloyl-2-amino-2-deoxy-D-glucitol at high concentration (above 10%, w/v) in water usually resulted in extremely viscous polymers and/or gels that were not completely soluble in water.

### Example 5.

### Polymerization kinetics of N-acryloyl-2-amino-2-deoxy-D-glucitol.

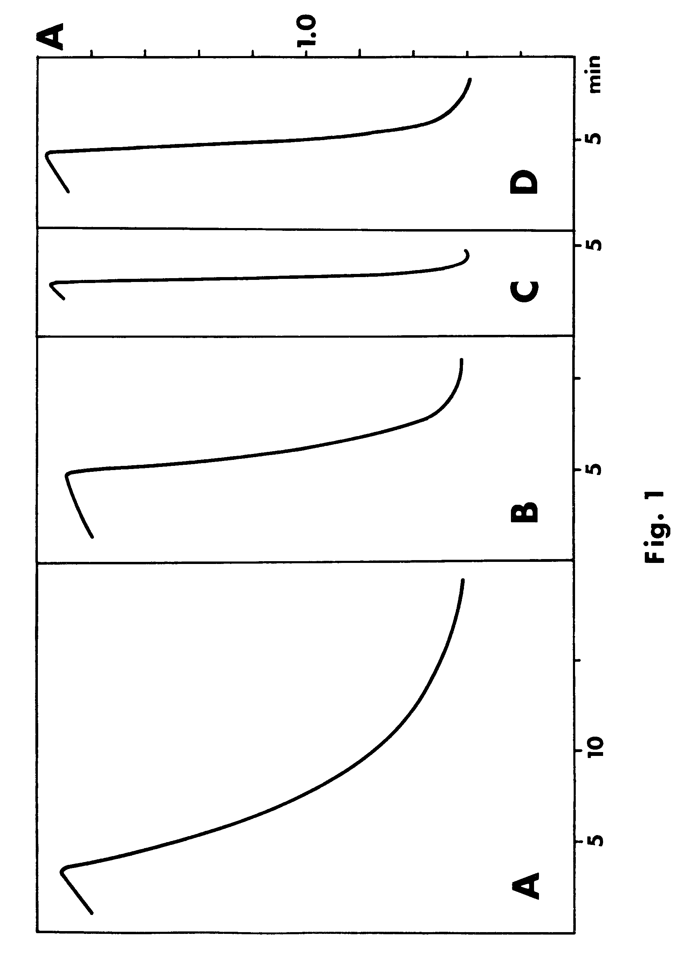

The polymerization kinetics (figure 1) of the title compound was compared to that of acrylamide (panel A) and N-acryloyl-tris(hydroxymethyl)aminomethane (panel B). The solutions were prepared which contained the same molar concentrations of the three monomers. The w/v concentrations were as follows: acrylamide 5%, NAT 12% and N-acryloyl-2-amino-2-deoxy-D-glucitol 16%. Polymerization of 1 ml portions was achieved by TEMED (1.5 fl) and ammonium persulfate (35 fl, 15mg/ml). The polymerizing solution was pipetted into quartz cuvette and overlaid with diisopropyl ether. The disappearance of the double bonds was followed spectrophotometrically in the UV region. The wavelengths were chosen so that the starting absorbancy was 1.8. As can be seen from figure 1, the title compound polymerizes much faster than the other two monomers (panel C). Even at the lower, 10% w/v, concentration the polymerization rate is higher than that of acrylamide and NAT (panel D).

**Note 3.** The higher polymerization rate indicates that the double bond of N-acryloyl-2-amino-2-deoxy-D-glucitol is more reactive than the same bond of the other two monomers and or that there are certain interactions between a growing polymer chain and the monomer, leading to an enhancement of the polymerization rate.

### Example 6.

### Transparency of the cross-linkedpoly-N-acryloyl-2-amino-2-deoxy-D-glucitol-N,N′-methylene-bis-acrylamide (Bis) gels.

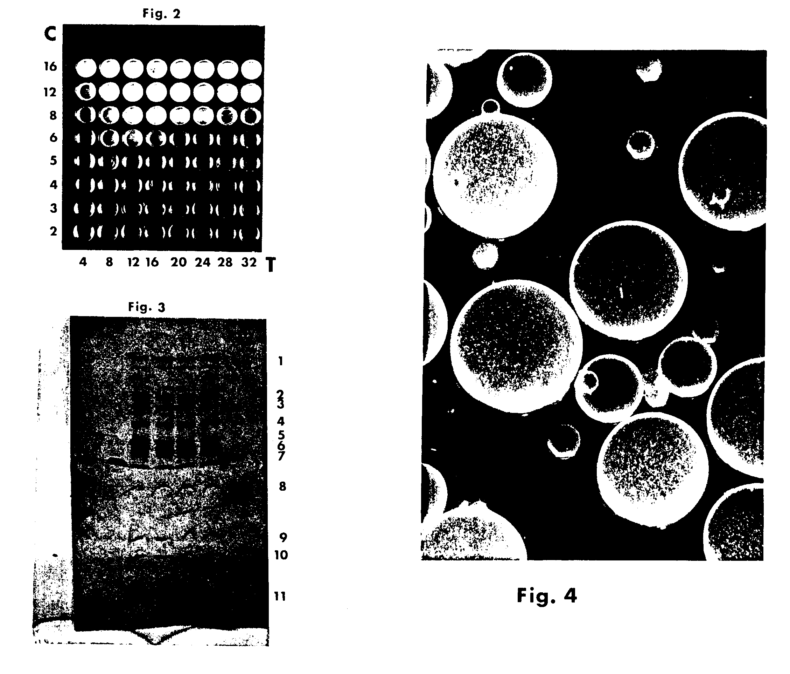

The title monomer and the cross-linker (Bis) were polymerized in 64 different combinations. The designation (T) represents total monomer concentration, which is the monomer and the cross-linker amount in grams per 100 ml of solution. The concentration of Bis (C) is expressed as a percentage to T. From the figure 2 one can notice the transparency of all gels containing 4 or less C. Some gels with 5 and 6 C are still transparent, whereas the gels having higher proportions of Bis became opaque. Since in most cases the transparent gels are used for electrophoresis and the opaque ones for chromatography, these results facilitate the choice of correct combinations.

### Example 7.

### Isoelectric focusing of proteins in a poly-N-acryloyl-2-amino-2-deoxy-D-glucitol-Bis gel.

The gel (T=7, C=3) was polymerized on a plastic sheet (Gel Bond), which contained polymerizable double bonds. In this way the gel was covalently linked to the plastic support. After polymerization the gel was washed three times with distilled water. It was then air-dried overnight at room temperature. The broad range (3.5-10) carrier ampholytes were introduced into the gel by the overlay technique, and the pre-focusing was done for 500 Vh. The standard proteins were applied to the gel, which was run for 2600 Vh. The following standard proteins can be seen after Coomassie Brilliant Blue staining (figure 3): 1, trypsinogen (pI 9.3); 2, lentil lectin basic band (pI 8.65); 3, lentil lectin middle band (pI 8.45); 4, lentil lectin acidic band (pI 8.15); 5, myoglobin basic band (pI 7.35); 6, myoglobin acidic band (pI 6.85); 7, human carbonic anhydrase (pI 6.55); 8, bovine carbonic anhydrase (pI 5.85); 9, beta-lactoglobulin A (pI 5.2); 10, soya-bean trypsin inhibitor (pI 4.55); 11, amyloglucosidase (pI 3,50).

### Example 8.

### Preparation of an opaque poly-N-acryloyl-2-amino-2-deoxy-D-glucitol-Bis gel in the form of beads.

A solution (4 ml) was prepared which contained the title monomer and the cross-linker (T=15, C=12), as well as 85 mg of poly-vinylalcohol (MW 15,000). Then, 24 ml of diethyl succinate containing 13 mg of Span 20 gas deaerated (degased), with magnetic stirring under high vacuum. This solution gas then transferred into an apparatus designed for suspension polymerization (Reference 5). The stirrer was inserted and a stream of nitrogen was continuously passing through the apparatus. After about 30 min, 18 »l of TEMED and 230 »l of ammonium persulfate (15 mg/ml) were added to the monomer solution, which was quickly, by means of a syringe, transferred into the apparatus. The suspension was stirred vigorously for about one hour. The formed beads were removed by gentle filtration and then washed with water by decantation. Figure 4 shows the beads prepared in this way.

### REFERENCES:

1. Kozulic, M., Kozulic, B., and Mosbach, K. (1987) Anal. Biochem. 163 506-512
2. Kozulic, B., Mosbach, K., and Pietrzak., M. (1988) Anal. Biochem. 170 478-484
3. Kozulic, B., and Mosbach, K. (1988) Patent Application, PCTEP88/00515
4. Whistler, R. L., Panzer, H.P., and Roberts H.J. (1961), J. Org. Chem. Vol. 26 p. 1583-1588
5. Arshady, R., and Ledwith, A. (1983) Reactive Polymers, 1 159-174

## Claims

1. A monomer polymerizable into reproducible gels suited for electrophoresis, which monomer has the formula: 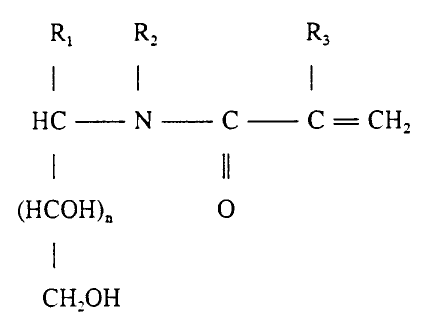 where R₁ is H, CH₂OH or (CHOH)ₘCH₂OH, m being 1 or 2;
R₂ is H or CH₃;
R₃ is H or CH₃; and
n is an integer of 2-4;
with the proviso that when n is 4 one of R₁ and R₂ cannot be H and when R₁ is H then n cannot be 2,
and contains substantially no other polymerizable compounds or polymerization inhibitors of a nature and in an amount sufficient to interfere with polymerization formation of said gels.

2. The monomer of claim 1, N-acryloyl-2-amino-2-deoxy-D-glucitol.

3. The monomer of claim 1, N-methacryloyl-2-amino-2-deoxy-D-glucitol.

4. The monomer of claim 1, N-acryloyl-N-methyl-1-amino-1-deoxy-D-glucitol.

5. The monomer of claim 1, N-methacryloyl-N-methyl-1-amino-1-deoxy-D-glucitol.

6. The monomer of claim 1, N-acryloyl-1-amino-1-deoxy-D-xylitol.

7. The monomer of claim 1, N-methacryloyl-1-amino-1-deoxy-D-xylitol.

8. The monomer of claim 1, N-acryloyl-N-methyl-1-amino-1-deoxy-D-xylitol.

9. The monomer of claim 1, N-methacryloyl-N-methy-1-amino-1-deoxy-D-xylitol.

10. A process for preparing an acrylic monomer of the formula: 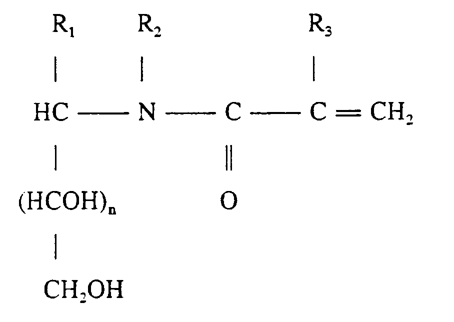 where R₁ is H, CH₂OH or (CHOH)ₘCH₂OH, m being 1 or 2;
R₂ is H or CH₃;
R₃ is H or CH₃; and
n is an integer of 1-4;
with the proviso that when R₁ is H then n cannot be 1
comprising reacting the aminodeoxy-sugar alcohol with acryloyl or methacryloyl chloride in a water-methylene chloride solvent two-phase mixture by alternate additions of the acid chloride and an alkali hydroxide at pH 7.5-9.5 and removing the charged by-products by a combination of an anionic and cationic ion exchanger.

11. A linear polymer which is the product of free-radical polymerization of an acrylic monomer of claim 1.

12. A cross-linked polymer as the product of free-radical polymerization of a cross-linker and an acrylic monomer, which monomer has the formula: 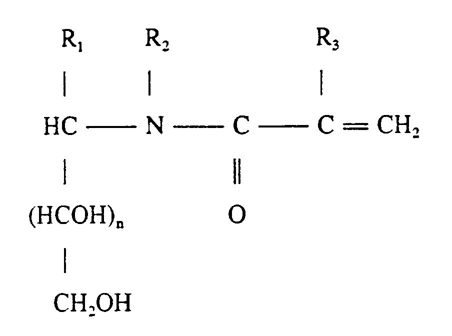 where R₁ is H, CH₂OH or (CHOH)ₘCH₂OH, m being 1 or 2;
R₂ is H or CH₃;
R₃ is H or CH₃; and
n is an integer of 2-4;
with the proviso that when R₁ is H then n cannot be 2,
and contains substantially no other polymerizable compounds or polymerization inhibitors of a nature or in an amount sufficient to interfere with polymerization formation of said polymer.

13. The polymer of claim 12, in form of an aqueous gel.

14. A method of electrophoresis comprising using as separation medium a gel of claim 13.

15. A method of isoelectric focusing comprising using as separation medium a gel of claim 13.

16. A method of chromatography comprising using as separation medium a polymer of claim 12.

## Patentansprüche

1. Ein Monomer, welches zu reproduzierbaren, für Elektrophorese geeigneten Gelen polymerisierbar ist, welches Monomer die Formel 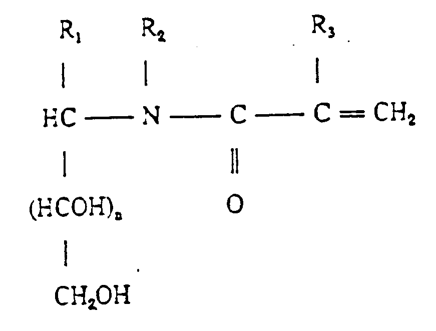 aufweist, worin gilt:
R₁ ist H, CH₂OH oder (CHOH)ₘCH₂OH, m ist 1 oder 2;
R₂ ist H oder CH₃;
R₃ ist H oder CH₃; und
n ist eine ganze Zahl von 2-4;
unter der Bedingung, dass wenn n=4 ist, entweder R₁ oder R₂ nicht H sein kann und dass wenn R₁ gleich H ist, dann n nicht 2 sein kann
und welches Monomer im Wesentlichen keine anderen polymerisierbaren Mischungen oder Polymerisationshemmstoffe enthält, welche von einer Art und einer genügenden Menge sind, dass sie die Polymerisationsform besagter Gele beeinträchtigen.

2. Das Monomer von Anspruch 1, N-Acryloyl-2-Amino-2-Deoxy-D-Glucitol.

3. Das Monomer von Anspruch 1, N-Methacryloyl-2-Amino-2-Deoxy-D-Glucitol.

4. Das Monomer von Anspruch 1, N-Acryloyl-N-Methyl-1-Amino-1-Deoxy-D-Glucitol.

5. Das Monomer von Anspruch 1, N-Methacryloyl-N-Methyl-1-Amino-1-Deoxy-D-Glucitol.

6. Das Monomer von Anspruch 1, N-Acryloyl-1-Amino-1-Deoxy-D-Xylitol.

7. Das Monomer von Anspruch 1, N-Methacryloyl-1-Amino-1-Deoxy-D-Xylitol.

8. Das Monomer von Anspruch 1, N-Acryloyl-N-Methyl-1-Amino-1-Deoxy-D-Xylitol.

9. Das Monomer von Anspruch 1, N-Methacryloyl-N-Methyl-1-Amino-1-Deoxy-D-Xylitol.

10. Ein Prozess zur Zubereitung eines Acrylmonomers mit der Formel 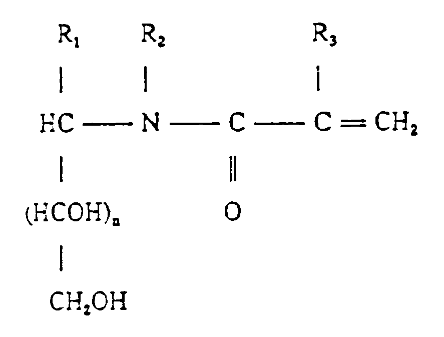 worin gilt:
R₁ ist H, CH₂OH oder (CHOH)ₘCH₂OH, m ist 1 oder 2;
R₂ ist H oder CH₃;
R₃ ist H oder CH₃; und
n ist eine ganze Zahl von 1-4;
unter der Bedingung, dass wenn R₁ gleich H ist, n nicht 1 sein kann,
welcher Prozess die Reaktion des Amino-Deoxy-Zucker-Alkohols mit Acryloyl oder Methacryloylchlorid in einer zweiphasigen Wasser-Methylenchloridlösung mittels einer wechselweisen Beimengung des sauren Chlorids und eines basischen Hydroxids bei pH 7.5-9.5 und mittels Entfernen der geladenen Nebenprodukte mit einer Kombination eines anionischen und kationischen Ionentauschers umfasst.

11. Ein lineares Polymer, welches das Erzeugnis einer Polymerisation durch freie Radikale eines Acrylmonomers von Anspruch 1 ist.

12. Ein vernetztes Polymer als Erzeugnis einer Polymerisation durch freie Radikale eines Vernetzers und eines Acrylmonomers, welches Monomer die Formel 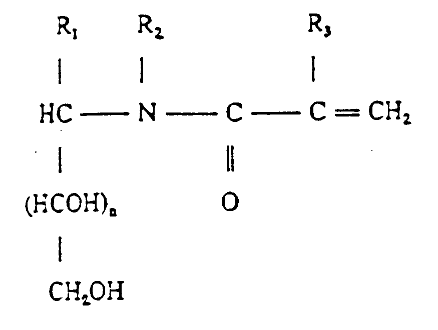 aufweist, worin gilt:
R₁ ist H, CH₂OH oder (CHOH)ₘCH₂OH, m ist 1 oder 2;
R₂ ist H oder CH₃;
R₃ ist H oder CH₃; und
n ist eine ganze Zahl von 2-4;
unter der Bedingung, dass wenn R₁ gleich H ist, n nicht 2 sein kann
und welches Monomer im Wesentlichen keine anderen polymerisierbaren Mischungen oder Polymerisationshemmstoffe enthält, welche von einer Art und einer genügenden Menge sind, dass sie die Polymerisationsformation besagten Polymers beeinträchtigen.

13. Das Polymer von Anspruch 12, in der Form eines wässrigen Gels.

14. Eine Elektrophoresemethode, welche den Gebrauch eines Gels von Anspruch 13 vorsieht.

15. Eine Methode zu isoelektrischen Fokussierung, welche den Gebrauch eines Gels von Anspruch 13 als Trennungsmittel vorsieht.

16. Eine Chromatographiemethode, welche den Gebrauch eines Polymers von Anspruch 12 als Trennungsmittel vorsieht.

## Revendications

1. Monomère polymérisable en gels reproductibles convenant pour l'électrophorèse, lequel monomère répond à la formule : 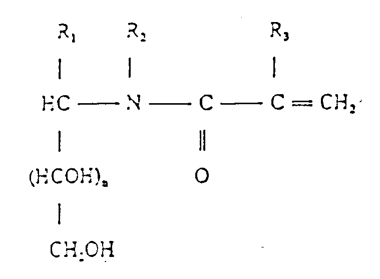 dans laquelle R₁ est H, CH₂OH ou (CHOH)ₘCH₂OH, m étant 1 ou 2 ;
R₂ est H ou CH₃ ;
R₃ est H ou CH₃ ; et
n est un entier de 2 à 4 ;
sous réserve que, lorsque n est 4, un des symboles R₁ et R₂ ne peut pas être H et que lorsque R₁ est H, n ne peut pas être 2,
et ne contient pratiquement aucun autre composé polymérisable ou inhibiteur de polymérisation dont la nature et la quantité soient telles qu'ils interfèrent avec la formation par polymérisation desdits gels.

2. Monomère selon la revendication 1, qui est le N-acryloyl-2-amino-2-désoxy-D-glucitol.

3. Monomère selon la revendication 1, qui est le N-méthacryloyl-2-amino-2-désoxy-D-glucitol.

4. Monomère selon la revendication 1, qui est le N-acryloyl-N-méthyl-1-amino-1-désoxy-D-glucitol.

5. Monomère selon la revendication 1, qui est le N-méthacryloyl-N-méthyl-1-amino-1-désoxy-D-glucitol.

6. Monomère selon la revendication 1, qui est le N-acryloyl-1-amino-1-désoxy-D-xylitol.

7. Monomère selon la revendication 1, qui est le N-méthacryloyl-1-amino-1-désoxy-D-xylitol.

8. Monomère selon la revendication 1, qui est le N-acryloyl-N-méthyl-1-amino-1-désoxy-D-xylitol.

9. Monomère selon la revendication 1, qui est le N-méthacryloyl-N-méthyl-1-amino-1-désoxy-D-xylitol.

10. Procédé de préparation d'un monomère acrylique répondant à la formule : 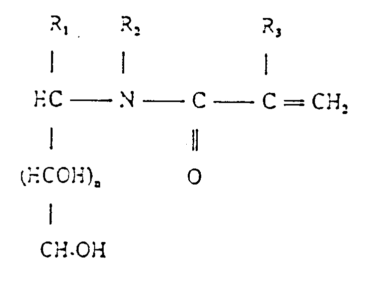 dans laquelle R₁ est H, CH₂OH ou (CHOH)ₘCH₂OH, m étant 1 ou 2 ;
R₂ est H ou CH₃ ;
R₃ est H ou CH₃ ; et
n est un entier de 1 à 4 ;
sous réserve que lorsque R₁ est H, n ne peut pas être 1,
comprenant le fait de faire réagir l'alcool d'aminodésoxy-sucre avec du chlorure d'acryloyle ou de méthacryloyle dans un mélange à deux phases eau-chlorure de méthylène comme solvant par des additions alternées du chlorure d'acide et d'un hydroxyde alcalin à pH 7,5 à 9,5, et d'éliminer les sous-produits chargés par une association d'échangeurs d'ions anioniques et cationiques.

11. Polymère linéaire qui est le produit de la polymérisation radicalaire d'un monomère acrylique selon la revendication 1.

12. Polymère réticulé en tant que produit de la polymérisation radicalaire d'un agent de réticulation et d'un monomère acrylique, lequel monomère répond à la formule : 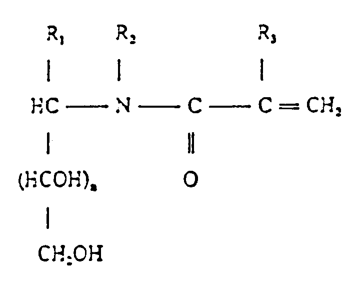 dans laquelle R₁ est H, CH₂OH ou (CHOH)ₘCH₂OH, m étant 1 ou 2 ;
R₂ est H ou CH₃ ;
R₃ est H ou CH₃ ; et
n est un entier de 2 à 4 ;
sous réserve que lorsque R₁ est H, n ne peut pas être 2,
et qui ne contient pratiquement aucun autre composé polymérisable ou inhibiteur de polymérisation dont la nature et la quantité soient telles qu'ils interfèrent avec la formation par polymérisation de ce polymère.

13. Polymère selon la revendication 12, sous la forme d'un gel aqueux.

14. Procédé d'électrophorèse comprenant l'utilisation comme milieu de séparation d'un gel selon la revendication 13.

15. Procédé de focalisation isoélectrique comprenant l'utilisation, comme milieu de séparation, d'un gel selon la revendication 13.

16. Procédé de chromatographie comprenant l'utilisation, comme milieu de séparation, d'un polymère selon la revendication 12.
